# EUROPEAN PATENT SPECIFICATION

(11) **EP 3 655 003 B1**
(45) Date of publication and mention of the grant of the patent: **07.05.2025**
(21) Application number: 18770072.9
(22) Date of filing: 18.07.2018
(51) Int. Cl.: A61K 36/23, A61K 36/258, A61P 25/28, A61K 31/355, A61K 31/385, A61K 33/04

(54) **COMPOSITIONS AND METHODS FOR THE TREATMENT AND PREVENTION OF COGNITIVE DECLINE AND PRESERVATION OF NEURONAL FUNCTION**
ZUSAMMENSETZUNGEN UND VERFAHREN ZUR BEHANDLUNG UND PRÄVENTION DES ABBAUS KOGNITIVER FÄHIGKEITEN UND BEWAHRUNG DER NEURONALEN FUNKTION
COMPOSITIONS ET MÉTHODES POUR LE TRAITEMENT ET LA PRÉVENTION DU DÉCLIN COGNITIF ET LA PRÉSERVATION DES FONCTIONS NEURONALES

(30) Priority: 18.07.2017 US 201762533926 P
(43) Date of publication of application: 27.05.2020
(73) Proprietor: Senescence Life Sciences Pte. Ltd., Singapore 408732 (SG)
(72) Inventor: WATSON, Shawn Nathan, Singapore 437903 (SG)
(74) Representative: D Young & Co LLP
(86) International application number: PCT/IB2018/055348
(87) International publication number: WO 2019/016731

(56) References cited:
- WO-A1-2012/143860
- US-A1- 2014 141 082
- US-A1- 2016 235 822
- US-B1- 6 733 797
- DATABASE GNPD [online] MINTEL; 4 October 2018 (2018-10-04), ANONYMOUS: "Dietary Supplement Capsules for Ages 30-55", XP055520331, retrieved from https://www.gnpd.com/sinatra/recordpage/6002813/ Database accession no. 6002813
- DATABASE GNPD [online] MINTEL; 4 October 2018 (2018-10-04), ANONYMOUS: "Dietary Supplement Capsules for Ages 55+", XP055520329, retrieved from https://www.gnpd.com/sinatra/recordpage/6002809/ Database accession no. 6002809
- N.N.: "Singapore-start-up-Senescence-targets-younger-demographic-with-new-cognitive-health-supplement", 18 April 2018 (2018-04-18), XP055520125, Retrieved from the Internet <URL:https://www.nutraingredients-asia.com/Article/2017/04/19/Singapore-start-up-Senescence-targets-younger-demographic-with-new-cognitive-health-supplement> [retrieved on 20181030]
- AMRITA TEJASVI: "REVIVE is a daily supplement to combat age-related cognitive decline", BIOSPECTRUM, 2 March 2017 (2017-03-02), Mumbai, XP055520170
- N.N.: "Singapore start-up Senescence targets younger demographic with", 18 April 2017 (2017-04-18), XP055520463, Retrieved from the Internet <URL:https://www.nutraingredients-asia.com/Article/2017/04/19/Singapore-start-up-Senescence-targets-younger-demographic-with-new-cognitive-health-supplement> [retrieved on 20181031]
- DATABASE GNPD [online] MINTEL; 4 October 2018 (2018-10-04), ANONYMOUS: "Dietary Supplement Capsules for Ages 30-55", XP055520331, retrieved from www.gnpd.com Database accession no. 6002813
- DATABASE GNPD [online] MINTEL; 4 October 2018 (2018-10-04), ANONYMOUS: "Dietary Supplement Capsules for Ages 55+", XP055520329, retrieved from www.gnpd.com Database accession no. 6002809

## Description

### Field of the Invention

The invention relates to compositions and compositions for use in treating cognitive decline.

### Background

Neurological decline associated with non-pathological aging is fast becoming a primary concern of Eastern and Western societies as we are beginning to feel the social, monetary and emotional pressures of an aging population. It is evident that the aging nervous system, whether invertebrate or vertebrate, suffers from a progressive deterioration of plastic components, most noticeably that of learning and memory. While this process likely has a diverse and multifactorial cellular and molecular foundation, one of the foremost mechanistic explanations is captured within the Free Radical Theory of Aging. The theory postulates that aging is the result of progressive pro-oxidative shifts in cellular redox states and consequential oxidation-dependent alterations in cell physiology.

While aging is the strongest predictor of numerous pathologies, some of which result in neuronal failure and atrophy, the fact remains that neuronal aging and cognitive decline are also observed in non-pathological iterations of aging. A large body of evidence indicates that oxidative stress is a prominent feature of both pathological and non-pathological forms of nervous system aging.¹⁻¹² If left unchecked, pro-oxidative shifts in cellular-redox states may cause cumulative damage and dysfunction to a variety of structures and processes within the cell, including the plasma membrane.^{8-10,13-15} Neurons, and in particular neuronal membranes, are highly susceptible to non-enzymatic lipid peroxidation, a form of radical attack, due to their post mitotic nature, high oxygen consumption, and elevated levels of poly-unsaturated fatty acids.¹³

The double bonds responsible for the unsaturation in lipids provide a means through which single electrons can become delocalized across 3 carbons. This facilitates a carbon centered free radical to form on the bis-allylic hydrogen.^{16,17} Due to the high concentration of molecular oxygen within neuronal membranes, the carbon centered free radical may quickly react to form a lipid peroxide radical within the hydrophobic domain of the bilayer. The negative charge on this peroxide may cause the acyl chain to migrate towards the surface (hydrophilic zone) of the bilayer. Movement of the acyl chain to the hydrophilic area may cause an increase in membrane rigidity as the ability of the phospholipids to move within and across layers becomes impaired. Thus, elevated levels of peroxidation may result in increased rigidity of the membrane, a phenomenon observed within both experimentally oxidized membranes as well as aged neuronal membranes.^{2,18,19} It is thought that due to the reactivity of the lipid peroxide, the radical can also go on to attack an adjacent poly-unsaturated fatty acid, thus starting what is known as a lipid peroxidation chain reaction.

Lipid peroxidation can lead to the production of deleterious lipid metabolites such as 4-hydroxynonenal (4-HNE), malondialdehyde (MDA), lipofuscin and many others.^{8,20-22} The build-up, and improper clearance of these and other pro-inflammatory metabolites has been observed in non-pathological neuronal aging, as well as implicated as a major factor related to the cognitive impairment associated with pathological neuronal diseases.²²⁻²⁸ A critical step linking membrane lipid peroxidation and the production of deleterious metabolites is the liberation of fatty acids from the membrane via the activity of phospholipase A₂ (PLA₂).

Following lipid peroxidation, PLA₂ is thought to function as a repair mechanism through the excision of oxidized fatty acids at the sn-2 position of phospholipids. Liberated fatty acids may then undergo glutathione-mediated reduction and consequential reincorporation into the membrane, if adequate reducing power is available, or remain as a peroxidized fatty acid.^{7,9,16,29-31} An up-regulation of PLA₂ has been shown to be a critical component in age-related neuronal changes in both pathological and non-pathological iterations of aging.^{5,6,10}

Of great interest, separate lines of research have shown free fatty acids can significantly alter (electro)physiological aspects of neuron(s) and network level functionalities through a variety of mechanisms.³²⁻³⁶ Free fatty acids have been shown to cause significant reductions in neuronal excitability primarily through alterations to both K⁺ and Ca²⁺ currents. In regards to K⁺, studies have found substantial free fatty acid effects on rectifying K⁺ currents, including those intricately linked to age-related changes in cellular excitability, like the *I*_{A} current ascribed to the fast-inactivating Kv4 K⁺ channels.³²⁻³⁶ Direct interactions involving the association of fatty acids with the inner cavity have been shown to result in a rapid inactivation of sustained K⁺ currents.^{32,37} Other studies show that peroxidized free fatty acids can function as oxidants and cause redox-related changes to cysteine residues within ion channels.^{33,38} Irrespective of target, slow, rapid and non-inactivating K⁺ currents have all been shown to be modulated by free fatty acids, some of which have also been closely linked to synaptic plasticity, including long term potentiation induction.³⁵

Fatty acids have also been shown to have a substantial capacity to inhibit low voltage activated Ca²⁺ channels (T-Type).^{35,39,40} These types of channels are key contributors to resting membrane potential and action potential generation, both important aspects of neuronal excitability control. Moreover, free fatty acids have been shown to have strong inhibitory effects on N-type Ca²⁺ channels, the primary channels responsible for pre-synaptic depolarization and neurotransmitter-related synaptic communication.⁴¹ It quickly becomes apparent that the neuromodulatory capabilities of free fatty acids are far reaching and can have significant consequences for neuronal (electro) physiology. If perturbed, the overwhelming amount of evidence may imply a substantive role for lipid peroxidation, PLA₂ activation and functional changes to the aging brain.

To date there are no comprehensive treatments specifically designed to ameliorate the deleterious consequences of age and/or lipid (per)oxidation-related changes in neuronal function. More specifically, no treatments are available which target the mechanism after the initial oxidative insult has occurred. There is a need in the art to identify compounds, compositions and methods for preventing cognitive decline and preserving neuronal function.

A further summary of the state of the art is provided by the references described herein and throughout.

Amrita Tejasvi: "REVIVE is a daily supplement to combat age-related cognitive decline", BIOSPECTRUM, 2 March 2017, XP055520170, Mumbai, discloses a composition comprising ginseng, *Centella,* tocopherol, selenium and lipoic acid for use in age-related cognitive decline.

Scattergood G, "Singapore start-up Senescence targets younger demographic with new cognitive health supplement", 18 April 2017, XP055520463, discloses a composition comprising ginseng, *Centella,* tocopherol, selenium and lipoic acid for use in age-related cognitive decline.

US 6733797 B1 discloses a neurochemical formulation comprising a supplement for improving function of neurons, improving memory and cognitive abilities, and reversing free radical damage caused by aging or neurodegenerative disease. The formulation comprises phosphoesters and antioxidants. Components may have antioxidant properties or enhance properties of other components.

US 2016/235822 A1 discloses dietary supplements, formulations, kits and methods for administration to an individual with type II diabetes or at risk of developing type II diabetes; formulations, kits and methods useful for treating, preventing, supporting, controlling, restoring, and/or maintaining blood sugar levels in individuals with type II diabetes or at risk of developing the same; and formulations, kits and methods useful for reducing and/or eliminating requirement of ex-vivo insulin administration in an individual diagnosed with type II diabetes.

US 2014/141082 A1 discloses compositions containing enriched and purified natural crocin and/or crocetin for prevention and/or treatment of cancers and other conditions and diseases. The compositions comprise mainly enriched or purified natural crocin or crocetin or combination of both and possible other active phytochemicals. The composition is used as a functional food, drink, dietary supplement, or therapeutic dosage to a human orally or through other appropriate way (parenteral, percutaneous, rectal, mucosal, intranasal or topical administration).

WO 2012/143860 A1 discloses a method of ameliorating cognitive decline or a related condition, comprising oral administration of an effective amount of one or more compositions of mixed phospholipids and use of a mixed phospholipid composition in the manufacture of an oral formulation for ameliorating cognitive decline or a related condition.

### Summary of the Invention

The present invention is defined in the appended claims. In one aspect, there is provided a composition in unit dose form comprising:
asiaticosides in an amount of 20-80 mg, wherein the asiaticosides are derived from *Centella asiatica* extract;
d-α-tocopherol succinate in an amount of 40-200 mg;
ginsenosides in an amount of 4-20 mg, wherein the ginsenosides are derived from Ginseng root extract;
L-selenomethionine in an amount of 10-50 mcg, and;
α-lipoic acid in an amount of 25-200 mg;
wherein the asiaticosides are present in an amount of 20% w/w or less of the composition.
In a further embodiment of the composition or compositions as outlined above, *Centella asiatica* extract is a *Centella asiatica* aqueous extract containing asiaticoside, or a *Centella asiatica* extract that inhibits phospholipase A₂.

In an embodiment of the composition or compositions as outlined above, the composition provides a synergistic effect on the treatment of cognitive decline compared to the treatment of cognitive decline with the individual components of the composition.

In a further embodiment of the composition or compositions as outlined above, *Centella asiatica* extract is from about 10 to about 50 % w/w of the composition.

In a further embodiment of the composition or compositions as outlined above, α-tocopherol is from about 10 to about 30 % w/w of the composition.

In a further embodiment of the composition or compositions as outlined above, ginseng extract is from about 10 to about 50 % w/w of the composition.

In a further embodiment of the composition or compositions as outlined above, α-lipoic acid is from about 1 to about 40 % w/w of the composition.

In a further embodiment of the present invention, there is provided a composition for use in treating or preventing cognitive decline in a subject in need thereof by administering the composition.

In a further embodiment of the composition for use as outlined above, the cognitive decline is normal age-related cognitive decline.

In yet a further embodiment of the composition for use as outlined above, the cognitive decline is loss of cognitive function, loss of memory, memory impairment, loss of short term memory, loss of intermediate term memory, loss of long term memory or loss of an ability to learn, store or recall information, loss of attention span, loss of language skills, loss of writing skills, loss of problem solving skills, loss of spatial processing, loss of focus, loss of emotional recognition accuracy/speed, loss of executive function accuracy/speed, or any combination thereof.

In yet a further embodiment of the composition for use as outlined above, the cognitive decline is from Alzheimer's disease, Lewy body dementia, vascular dementia, Parkinson's disease-associated dementia, Huntington's disease-associated dementia, AIDS-associated dementia, benign senile forgetfulness, Down's syndrome-associated dementia, multi-infarct dementia, multiple sclerosis, tardive dyskinesia, Wernicke-Korsakoff syndrome or alcoholism-associated dementia.

In an embodiment of the composition for use as outlined above, the composition is administered to the subject simultaneously, sequentially or in combination with a pharmacological agent.

In another embodiment of the composition for use as outlined above, the pharmacological agent is for the treatment of Alzheimer's disease, Lewy body dementia, vascular dementia, Parkinson's disease-associated dementia, Huntington's disease-associated dementia, AIDS-associated dementia, benign senile forgetfulness, Down's syndrome-associated dementia, multi-infarct dementia, multiple sclerosis, tardive dyskinesia, Wernicke-Korsakoff syndrome or alcoholism-associated dementia.

In an embodiment of the composition for use as outlined above, the composition is in the form of a pill, capsule, liquid, syrup, powder, cream, ointment, gel, or paste.

In an embodiment of the composition for use as outlined above, the composition is for daily administration.

In an embodiment of the present invention, the composition is a food product comprising the composition.

In an embodiment of the food product or products as outlined above, the composition is incorporated into, but not limited to an energy bar, oatmeal, cereal or yogurt.

In an embodiment of the present invention, the composition is a beverage product comprising the composition.

In an embodiment of the beverage product or products as outlined above, the composition is incorporated into, but not limited to an energy shake, smoothie, tea, coffee or flavoured water.

In a more preferred embodiment, the composition in unit dosage form comprises about 50 mg or 100 mg α-lipoic acid, about 25 mcg L-selenomethionine, about 83 mg or 125 mg d-α-tocopherol succinate, about 200 mg or 175 mg *Centella asiatica* whole plant extract providing about 40 mg or 35 mg asiaticosides, respectively; and 175 mg or 150 mg Panax Ginseng extract comprising about 8.75 mg and 7.5 mg ginsenosides, respectively.

It is further contemplated that the compositions or formulations as described herein and throughout may further comprise one or more pharmaceutically acceptable excipients, for example, but not limited to microcrystalline cellulose, magnesium stearate, silicon dioxide or a combination thereof. Other excipients also may be included.

The present invention also provides a composition or formulation as described herein and throughout for use in improving cognitive function or treating and/or preventing cognitive decline in a subject in need thereof.

Also contemplated by the present invention is a composition for use in treating or preventing cognitive decline in a subject in need thereof by administering a composition or formulation as described herein and throughout to the subject. The cognitive decline may be normal age-related cognitive decline, for example, but not limited to loss of cognitive function, loss of memory, memory impairment, loss of short term memory, loss of intermediate term memory, loss of long term memory or loss of an ability to learn, store or recall information, loss of attention span, loss of language skills, loss of writing skills, loss of problem solving skills, loss of spatial processing, loss of focus, loss of emotional recognition accuracy/speed, loss of executive function accuracy/speed, or any combination thereof.

In a further embodiment, the cognitive decline is from a degenerative disease or disorder, for example, but not limited to Alzheimer's disease, Lewy body dementia, vascular dementia, Parkinson's disease-associated dementia, Huntington's disease-associated dementia, AIDS-associated dementia, benign senile forgetfulness, Down's syndrome-associated dementia, multi-infarct dementia, multiple sclerosis, tardive dyskinesia, Wernicke-Korsakoff syndrome or alcoholism-associated dementia.

In a further embodiment, the composition is as described herein but is not a composition comprising 125mg (150IU) d-alpha-tocopherol succinate, 75mg alpha-lipoic acid, 175 mg *Centella asiatica* whole plant extract comprising 40% asiaticosides, 175mg Panax Ginseng stem and leaves extract comprising 5% ginsenosides and 25mcg L-selenomethionine.
The present invention also contemplates a method of making any composition or formulation comprising combining d-alpha-tocopherol succinate, alpha-lipoic acid, *centella asiatica* extract, panax ginseng root extract and L-selenomethionine, wherein *centella asiatica* extract and panax ginseng root extract are derived from water-ethanol extractions. The method may further comprise packaging the composition in a capsule with microcrystalline cellulose, magnesium stearate and silicon dioxide. However, a variety of other methodologies as is known in the art also may be employed.

### Brief Description of the Figures:

Figure 1 shows results of verbal learning accuracy tests (verbal learning number correct) on human subjects during clinical trials at baseline and following 2 and 4 months dosing with compositions of the instant invention.
Figure 2 shows results of impulse control response speed tests (go-no-go response time totals) on human subjects during clinical trials at baseline and following 2 and 4 months dosing with compositions of the instant invention.
Figure 3 shows results of cognitive processing speed tests (Stroop response times) on human subjects during clinical trials at baseline and following 2 and 4 months dosing with compositions of the instant invention.
Figure 4 shows results of emotional recognition speed tests (emotional recognition time totals) on human subjects during clinical trials at baseline and following 2 and 4 months dosing with compositions of the instant invention.
Figure 5 shows results of cognitive processing accuracy tests (Stroop number correct) on human subjects during clinical trials at baseline and following 2 and 4 months dosing with compositions of the instant invention.
Figure 6 shows results of emotional recognition accuracy tests (emotional recognition number correct) on human subjects during clinical trials at baseline and following 2 and 4 months dosing with compositions of the instant invention.
Figure 7 shows results of executive function speed tests (trail making times) on human subjects during clinical trials at baseline and following 2 and 4 months dosing with compositions of the instant invention.
Figure 8 shows results of spatial processing speed tests (maze trail times) on human subjects during clinical trials at baseline and following 2 and 4 months dosing with compositions of the instant invention.
Figure 9 shows results of attention accuracy tests (digit span number correct) on human subjects during clinical trials at baseline and following 2 and 4 months dosing with compositions of the instant invention.
Figure 10 shows results of verbal learning accuracy tests (verbal learning number correct) on human subjects during clinical trials at baseline and following 2 and 4 months dosing with compositions of the instant invention.
Figure 11 shows results of verbal learning accuracy tests (verbal learning number incorrect) on human subjects during clinical trials at baseline and following 2 and 4 months dosing with compositions of the instant invention.
Figure 12 shows results of processing speed tests (Stroop response times) on human subjects during clinical trials at baseline and following 2 and 4 months dosing with compositions of the instant invention.
Figure 13 shows results of processing accuracy tests (Stroop test number correct) on human subjects during clinical trials at baseline and following 2 and 4 months dosing with compositions of the instant invention.
Figure 14 shows results of working memory tests (N-Back response total times) on human subjects during clinical trials at baseline and following 2 and 4 months dosing with compositions of the instant invention.
Figure 15 shows results of emotional recognition speed tests (emotional recognition response total times) on human subjects during clinical trials at baseline and following 2 and 4 months dosing with compositions of the instant invention.
Figure 16 shows results of emotional recognition accuracy tests (emotional recognition number correct) on human subjects during clinical trials at baseline and following 2 and 4 months dosing with compositions of the instant invention.
Figure 17 shows results of emotional recognition accuracy tests (emotional recognition number incorrect) on human subjects during clinical trials at baseline and following 2 and 4 months dosing with compositions of the instant invention.
Figure 18 shows results of executive function speed tests (trail making total times) on human subjects during clinical trials at baseline and following 2 and 4 months dosing with compositions of the instant invention.
Figure 19 shows results of executive function accuracy tests (trail making number of incorrect) on human subjects during clinical trials at baseline and following 2 and 4 months dosing with compositions of the instant invention.
Figure 20 shows results of spatial processing speed tests (maze trail total times) on human subjects during clinical trials at baseline and following 2 and 4 months dosing with compositions of the instant invention.
Figure 21 shows results of spatial processing accuracy tests (maze trail number incorrect) on human subjects during clinical trials at baseline and following 2 and 4 months dosing with compositions of the instant invention.
Figure 22 shows results of attention accuracy tests (complex figure number correct) on human subjects during clinical trials at baseline and following 2 and 4 months dosing with compositions of the instant invention.
Figure 23 shows results of attention accuracy tests (complex figure number incorrect) on human subjects during clinical trials at baseline and following 2 and 4 months dosing with compositions of the instant invention.

### Detailed Description

Described herein are compositions for preventing cognitive decline and methods for treating cognitive decline. Also described herein are compositions and methods for preserving neuronal function. It will be appreciated that the compositions, methods and embodiments described herein are for illustrative purposes intended for those skilled in the art All references to embodiments or examples throughout the disclosure should be considered a reference to an illustrative embodiment or an illustrative example. As noted above, the scope of the present invention is defined in the appended claims. In one aspect, there is provided a composition in unit dose form comprising:
asiaticosides in an amount of 20-80 mg, wherein the asiaticosides are derived from *Centella asiatica* extract;
d-α-tocopherol succinate in an amount of 40-200 mg;
ginsenosides in an amount of 4-20 mg, wherein the ginsenosides are derived from Ginseng root extract;
L-selenomethionine in an amount of 10-50 mcg, and;
α-lipoic acid in an amount of 25-200 mg;
wherein the asiaticosides are present in an amount of 20% w/w or less of the composition.

Described herein, without being claimed, is a composition for preventing and/or treating cognitive decline, the composition comprising:
a phospholipase A₂ inhibitor;
a lipophilic antioxidant;
a glutathione peroxidase enhancer; and
a glutathione level enhancer.

Also described herein, without being claimed, is a composition for preserving neuronal function, the composition comprising:
a phospholipase A₂ inhibitor;
a lipophilic antioxidant;
a glutathione peroxidase enhancer; and
a glutathione level enhancer.

In the context of the present disclosure, by the term "a phospholipase A₂ inhibitor" it is meant an agent, chemical or protein that reduces, inhibits or suppresses the activity of PLA₂. An agent, chemical or protein may be tested for its ability to reduce, inhibit or suppress PLA₂ activity in any assay known in the art, for example, but not limited to Invitrogen^{™} EnzChek^{®} Phospholipase A₂ Assay Kit. In this assay, an agent, chemical or protein is considered a PLA₂ inhibitor if it reduces the activity of PLA₂ by 10 to 100%, for example, but not limited to 10, 20, 30, 40, 50, 60, 70, 80, 90 or 100% when compared to a negative control. One representative example of a PLA₂ inhibitor which is not meant to be limiting in any manner is *Centella asiatica,* also known as Gotu kola. It will also be appreciated that an extract of *Centella asiatica* may be used in the above described composition. The extract may be obtained from the aqueous extract, alcohol extract, acetone extract or a combination thereof, of leaves or edible plant parts.⁷¹ The whole plant of *Centella asiatica* can be also used. The extract and whole plant may contain pentacycylic triterpenoids, for example, but not limited to asiaticoside. Other representative examples of PLA₂ inhibitors are ginseng or an extract thereof, fatty acid trifluoromethyl ketone, methyl arachidonyl fluorophosphonate, bromoenol lactone, 1,3-disubstituted propan-2-ones polyfluoroalkyl ketones, stearidonic acid, curcumin or an extract thereof, and aristolochic acid. The composition in unit dosage form of the present invention comprises asiaticosides derived from *Centella asiatica* extract.

In the context of the present disclosure, by the term "lipophilic antioxidant" it is meant an agent, chemical or protein that inhibits the oxidation of membrane lipids. The lipophilic antioxidant also includes a hydrophobic (lipophilic) region, for example, an alkyl chain. An agent, chemical or protein may be tested for its antioxidant activity by any assay known in the art, for example, but not limited to Sigma-Aldrich^{®} Antioxidant Assay Kit. In this assay, an agent, chemical or protein is considered an antioxidant if it possesses 10 to 100% antioxidant activity, for example, but not limited to 10, 20, 30, 40, 50, 60, 70, 80, 90 or 100% when compared to a positive control. One representative example of a lipophilic antioxidant is tocopherol. It will be appreciated that tocopherols include natural or synthetic α-, β-, γ- and δ-tocopherols, with α-tocopherol being the most active antioxidant. A derivative of α-tocopherol, such as α-tocopherol succinate, may also be contemplated. The composition in unit dosage form of the present invention comprises d-α-tocopherol succinate.

In the context of the present disclosure, by the term "a glutathione peroxidase enhancer" it is meant an agent, chemical or protein that enhances or increases the activity of glutathione peroxidase. An agent, chemical or protein may be tested for its ability to enhance or increase glutathione peroxidase activity in any glutathione peroxidase assay known in the art, for example, but not limited to Sigma-Aldrich^{®} Glutathione Peroxidase Assay Kit. In this assay, an agent, chemical or protein is considered a glutathione peroxidase enhancer if it increases the activity of glutathione peroxidase by 10 to 100%, for example, but not limited to 10, 20, 30, 40, 50, 60, 70, 80, 90 or 100% when compared to a negative control. One representative example of a glutathione peroxidase enhancer is ginseng. It will be appreciated that ginseng can be obtained from an aqueous extract, alcohol extract or a combination thereof of ginseng root, stem and leaf or the whole plant itself may be used, for example, in a powdered or other form. Other representative examples of glutathione peroxidase enhancers are selenocysteine, selenium and cysteine. The composition in unit dosage form of the present invention comprises ginsengosides derived from Ginseng root extract and L-selenomethionine.

In the context of the present disclosure, by the term "a glutathione level enhancer" it is meant an agent, chemical or protein that enhances or increases the level of glutathione. An agent, chemical or protein may be tested for its ability to increase glutathione levels in any glutathione assay known in the art, for example, but not limited to Sigma-Aldrich^{®} Glutathione Peroxidase Assay Kit. In preparation for this assay, cells may be incubated with the compound of interest and then lysed in accordance with procedures and methods commonly known in the art. The cell lysate may be used as a substrate in the assay to determine glutathione levels. In this assay, an agent, chemical or protein is considered a glutathione level enhancer if it increases the level of glutathione by 10 to 100%, for example, but not limited to 10, 20, 30, 40, 50, 60, 70, 80, 90 or 100% when compared to a negative control. One representative example of a glutathione level enhancer is α-lipoic acid. Another representative example of a glutathione level enhancer i*s N-*acetylcysteine. The composition in unit dosage form of the present invention comprises α-lipoic acid.

A composition described herein without being claimed comprises from about 1 to about 10,000 mg of phospholipase A₂ inhibitor, for example, but not limited to 1, 10, 20, 50, 100, 200, 300, 400, 500, 1000, 2000, 3000, 4000, 5000, 6000, 7000, 8000, 9000 or 10,000 mg of phospholipase A₂ inhibitor. As further described herein, such a composition may comprise a range of amounts as defined by any two of the values listed above or any two amounts therein between. The phospholipase A₂ inhibitor of the described compositions is preferably *Centella asiatica* or an extract thereof. The composition in unit dosage form of the present invention comprises a phospholipase A₂ inhibitor that is asiaticosides in an amount of 20-80 mg, wherein the asiaticosides are derived from *Centella asiatica* extract.

A composition described herein without being claimed comprises from about 1 to about 10,000 mg of lipophilic antioxidant, for example, but not limited to 1, 10, 20, 50, 100, 200, 300, 400, 500, 1000, 2000, 3000, 4000, 5000, 6000, 7000, 8000, 9000 or 10,000 mg of lipophilic antioxidant. As further described herein, such a composition may comprise a range of amounts as defined by any two of the values listed above or any two amounts therein between. The lipophilic antioxidant of the described compositions is preferably α-tocopherol or a derivative thereof. The composition in unit dosage form of the present invention comprises a lipophilic antioxidant that is d-α-tocopherol succinate in an amount of 40-200 mg.

A composition described herein without being claimed comprises from about 1 to about 10,000 mg of a glutathione peroxidase enhancer, for example, but not limited to 1, 10, 20, 50, 100, 200, 300, 400, 500, 1000, 2000, 3000, 4000, 5000, 6000, 7000, 8000, 9000 or 10,000 mg of a glutathione peroxidase enhancer. As further described herein, such a composition may comprise a range of amounts as defined by any two of the values listed above or any two amounts therein between. The glutathione peroxidase enhancer of the described compositions is preferably ginseng or an extract thereof, selenocysteine or a combination thereof. The composition in unit dosage form of the present invention comprises a glutathione enhancer that is ginsenosides in an amount of 4-20 mg, wherein the ginsenosides are derived from Ginseng root extract, and L-selenomethionine in an amount of 10-50 mcg.

A composition described herein without being claimed comprises from about 1 to about 10,000 mg of a glutathione level enhancer, for example, but not limited to 1, 10, 20, 50, 100, 200, 300, 400, 500, 1000, 2000, 3000, 4000, 5000, 6000, 7000, 8000, 9000 or 10,000 mg of a glutathione level enhancer. As further described herein, such a composition may comprise a range of amounts as defined by any two of the values listed above or any two amounts therein between. The glutathione level enhancer of the described compositions is preferably α-lipoic acid. The composition in unit dosage form of the present invention comprises a glutathione level enhancer that is α-lipoic acid in an amount of 25-200 mg

A composition described herein comprises *Centella asiatica* or an extract thereof, α-tocopherol or a derivative thereof, ginseng or an extract thereof, selenocysteine and α-lipoic acid. Extracts of *Centella asiatica* and ginseng may be performed by water/ethanol extraction via homogenation or by any other suitable solvent system and/or extraction process known in the art. In a preferred embodiment, the extraction process for *Centella asiatica* and ginseng is a water/ethanol extraction process and produces an extract comprising about 20% w/w asiaticosides and about 5% w/w total ginsenosides, respectively when dried. However, the present invention also contemplates embodiments wherein the extraction process results in 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40% w/w or higher asiaticosides or ginsenosides when dried. As described herein, the amount of asiaticosides or ginsenosides may also be defined by a range by any two of the values recited or any numbers therein between. The amount ranges of the asiaticosides and ginsengosides of the composition in unit dosage form of the present invention are further defined in the appended claim set.

A composition described herein without being claimed comprises from about 1 to about 10,000 mg of *Centella asiatica* or an extract thereof, for example, but not limited to 1, 10, 20, 50, 100, 200, 300, 400, 500, 1000, 2000, 3000, 4000, 5000, 6000, 7000, 8000, 9000 or 10,000 mg of *Centella asiatica* or an extract thereof. As further described herein, such a composition may comprise a range of amounts as defined by any two of the values listed above or any two amounts therein between.

A composition described herein without being claimed comprises from about 1 to about 10,000 mg of α-tocopherol or a derivative thereof, for example, but not limited to 1, 10, 20, 50, 100, 200, 300, 400, 500, 1000, 2000, 3000, 4000, 5000, 6000, 7000, 8000, 9000 or 10,000 mg of α-tocopherol or a derivative thereof. As further described herein, such a composition may comprise a range of amounts as defined by any two of the values listed or any two amounts therein between.

A composition described herein without being claimed comprises from about 1 to about 10,000 mg of ginseng or an extract thereof, for example, but not limited to 1, 10, 20, 50, 100, 200, 300, 400, 500, 1000, 2000, 3000, 4000, 5000, 6000, 7000, 8000, 9000 or 10,000 mg of ginseng or an extract thereof. As further described herein, such a composition may comprise a range of amounts as defined by any two of the values listed above or any two amounts therein between.

A composition described herein without being claimed comprises from about 0.001 to about 0.5 mg of selenocysteine, for example, but not limited to 0.001, 0.005, 0.01, 0.015, 0.02, 0.03, 0.05, 0.07, 0.1, 0.2, 0.3, 0.4 or 0.5 mg of selenocysteine. As further described herein, such a composition may comprise a range of amounts as defined by any two of the values listed above or any two amounts therein between. The present disclosure also contemplates replacing selenocysteine in any passage or composition described herein with selenomethionine and vice-versa unless clearly indicated otherwise.

A composition described herein without being claimed comprises from about 1 to about 10,000 mg of α-lipoic acid, for example, but not limited to 1, 10, 20, 50, 100, 200, 300, 400, 500, 1000, 2000, 3000, 4000, 5000, 6000, 7000, 8000, 9000 or 10,000 mg of α-lipoic acid. As further described herein, such a composition may comprise a range of amounts as defined by any two of the values listed above or any two amounts therein between.

One example of the composition, as further defined in the appended claim set, comprises from about 10 to about 50 % w/w of *Centella asiatica* or an extract thereof, for example, but not limited to 10, 20, 30, 40 or 50 % w/w of *Centella asiatica* or an extract thereof.

A composition described herein without being claimed comprises from about 10 to 30 % w/w of α-tocopherol or a derivative thereof, for example, but not limited to 10, 20 or 30 % w/w of α-tocopherol or a derivative thereof.

Yet another example of the composition, as further defined in the appended claim set, comprises from about 10 to 50 % w/w of ginseng or an extract thereof, for example, but not limited to 10, 20, 30, 40 or 50 % w/w of ginseng or an extract thereof.

A composition described herein without being claimed comprises from about 0.0001 to 0.001 % w/w of selenocysteine, for example, but not limited to 0.0001, 0.0002, 0.0005, 0.0007 or 0.001 % w/w of selenocysteine.

A further example of the composition, as further defined in the appended claim set, comprises from about 1 to 40 % w/w of α-lipoic acid, for example, but not limited to 1, 2, 5, 7, 10, 20, 30 or 40 % w/w of α-lipoic acid.

A composition described herein without being claimed comprises the following ratio of components: *Centella asiatica* or an extract thereof : α-tocopherol or a derivative thereof : ginseng or an extract thereof : selenocysteine : α-lipoic acid of about 5000-10000 : 3000-7000 : 5000-10000 : 1-5 : 1000-6000 by weight.

A composition described herein without being claimed may comprise the ratio of *Centella asiatica* or an extract thereof : α-tocopherol or a derivative thereof : ginseng or an extract thereof : selenocysteine : α-lipoic acid of about 7840 : 4900 : 7840 : 1 : 2960 by weight.

It will be appreciated that the phospholipase A₂ inhibitor, the lipophilic antioxidant, the glutathione peroxidase enhancer and the glutathione level enhancer are preferably compounds that have no cytotoxicity or low cytotoxicity for cells or humans when the composition comprising these compounds is administered at the dosages described herein.

Also described herein is a method for preserving, improving or increasing neuronal function in a neuronal cell by treating the neuronal cell with the composition as described herein. The neuronal cell may be treated *in-vitro* or *in-vivo.*

In the context of the present disclosure, by the term "neuronal function" it is meant any physiological parameter of normal neuronal activity. This can include but is not limited to: neuronal electrophysiology, synaptic efficacy, neuron shape/size, metabolite composition and/or energy usage. It will be appreciated that neuronal function may be preserved by decreasing lipid peroxidation. Neuronal function also may be preserved by maintaining, increasing or improving neuronal excitability. The increase or preservation of neuronal function can be measured by assessing common metrics used in electrophysiological assessments. These include but are not limited to: action potential frequency and shape, resting membrane potential, membrane resistance and/or rheobase measurements. Described herein is a method for treating or preventing cognitive decline in a subject in need thereof by administering the composition to the subject. One aspect of the present invention provides a composition for usein improving cognitive function or treating and/or preventing cognitive decline in a subject in need thereof as defined in the appended claims.

Without wishing to be bound by theory or limiting in any manner, cognitive decline refers to the typical cognitive decline that commonly becomes most apparent in individuals over the age of 40. Cognitive decline in a subject may include varying levels of loss of cognitive function, loss of memory, memory impairment, loss of short term memory, loss of intermediate term memory, loss of long term memory, loss of an ability to learn, store or recall information or loss of spatial processing, loss of focus, loss of emotional recognition accuracy/speed, loss of executive function accuracy/speed, or any combination thereof. These symptoms may occur at a minimal level, commonly associated with the natural aging process, or may occur with more severity as the individual ages.

The symptoms may be determined based on the individual's own experiences. The symptoms may also be observed by family and friends of the individual. Some examples of symptoms that can indicate cognitive decline are, without limitation, forgetfulness, especially for important events such as appointments or social engagements, misplacing objects, losing train of thought or thread of a conversation, book or movie, difficulty in finding words, repeatedly asking the same questions, inability to follow directions, disorientation as to the date or time of day, not recognizing familiar people, feeling overwhelmed by making decisions, having difficulty planning steps to accomplish a task or interpreting instructions, having difficulty finding the way around familiar environments, impulsiveness, showing poor judgment and slowed speed of cognitive processing.

In a further embodiment, it is contemplated that cognitive decline may also be related to a disease state such as, but not limited to, Alzheimer's disease, Lewy body dementia, vascular dementia, Parkinson's disease-associated dementia, Huntington's disease-associated dementia, AIDS-associated dementia, benign senile forgetfulness, Down's syndrome-associated dementia, multi-infarct dementia, multiple sclerosis, tardive dyskinesia, Wernicke-Korsakoff syndrome or alcoholism-associated dementia.

The subject may be suspected of having cognitive decline by being evaluated based on the symptoms stated above or the results of cognitive tests. It is appreciated that cognitive decline does not need to be diagnosed by a physician and may be determined by the subject's own experiences.

Also described is a method for preserving neuronal function in a subject by administering the composition to the subject. It is appreciated that improvements and/or preservation of neuronal function may be determined clinically or subjectively by the subject or a person observing the subject over a period of time.

As described herein without being claimed, an oral formulation of a composition may comprise from about 1 mg to about 10 g phospholipase A₂ inhibitor, from about 1 mg to about 10 g lipophilic antioxidant, from about 0.001 mg to about 10 g glutathione peroxidase enhancer and from about 1 mg to about 10 g glutathione level enhancer.

As described herein without being claimed, an oral formulation of a composition may comprise, for example, about 200 mg phospholipase A₂ inhibitor, about 125 mg lipophilic antioxidant, about 200 mg glutathione peroxidase enhancer and about 76 mg glutathione level enhancer.

As described herein without being claimed, an oral formulation of a composition may comprise from about 1 mg to about 10 g *Centella asiatica* or an extract thereof, from about 1 mg to about 10 g α-tocopherol or a derivative thereof, from about 1 mg to about 10 g ginseng or an extract thereof, from about 0.001 to about 10 mg selenocysteine and from about 1 mg to about 10 g α-lipoic acid.

**Table 1**

| Composition Component | Amount (mg) |
|---|---|
| *Centella asiatica* or an extract thereof | 200.0000 |
| α-Tocopherol or a derivative thereof | 125.0000 |
| Ginseng or an extract thereof | 200.0000 |
| Selenocysteine | 0.0255 |
| α-Lipoic acid | 76 |

In an example as shown in **Table 1** (not claimed), an oral formation of a described composition may comprise about 200 mg *Centella asiatica* or an extract thereof, about 125 mg α-tocopherol or a derivative thereof, about 200 mg ginseng or an extract thereof, about 0.025 mg selenocysteine and about 76 mg α-lipoic acid.

It will be appreciated that the composition may be in the form of a pill, capsule, liquid, syrup, powder, cream, ointment, gel, paste, or any other formulation which will enable the delivery of the composition for example, but not limited to, into the body of the subject.

In one embodiment, a dose of the composition may be taken once a day or more, for example, 1, 2, 3, 4, 5, 6, 7, 8, 9 or 10 times daily. Doses of the composition taken at different times of the day may be in the same or varying amounts. Without being limiting, an example of a dosing schedule may be a dose of 0.5 g in the morning, a dose of 0.25 g at lunch and a dose of 1 g in the evening. In a preferred embodiment, which is not meant to be limiting, the composition is taken daily.

It is also contemplated that a food or beverage product may comprise the composition as described herein. For example, the composition may be incorporated into, for example, energy bars, oatmeal, cereal or yogurt. The composition may also be incorporated into beverages, for example, protein shakes, smoothies, tea, coffee, sports drinks or flavoured water.

### Example 1

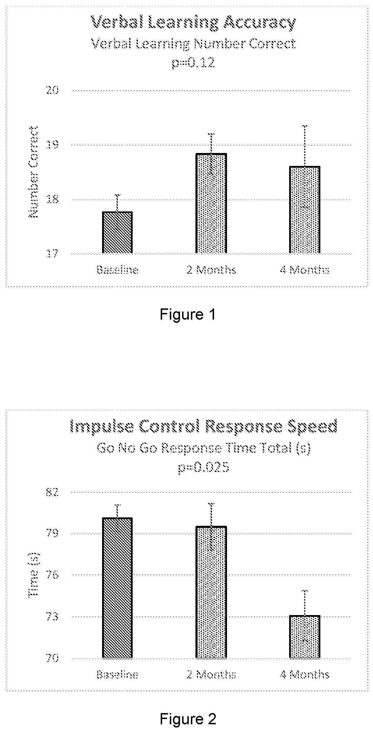

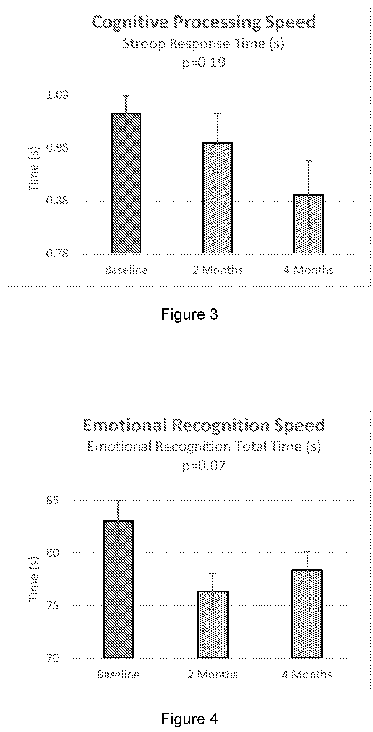

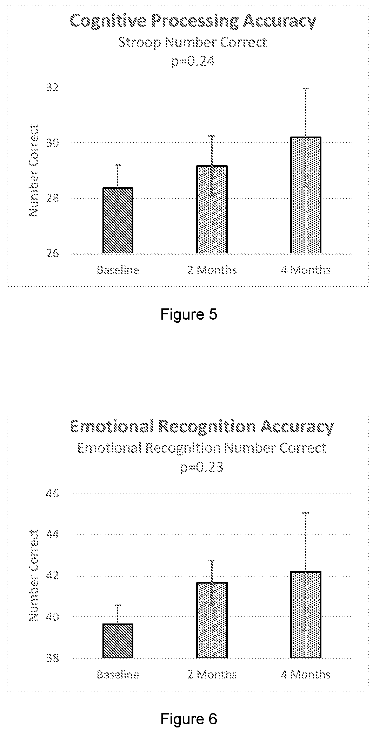

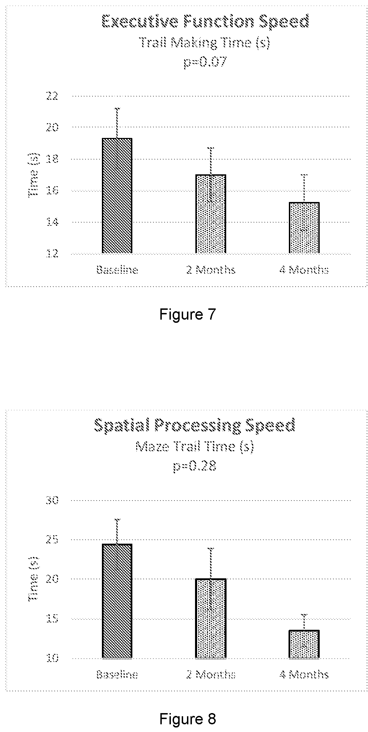

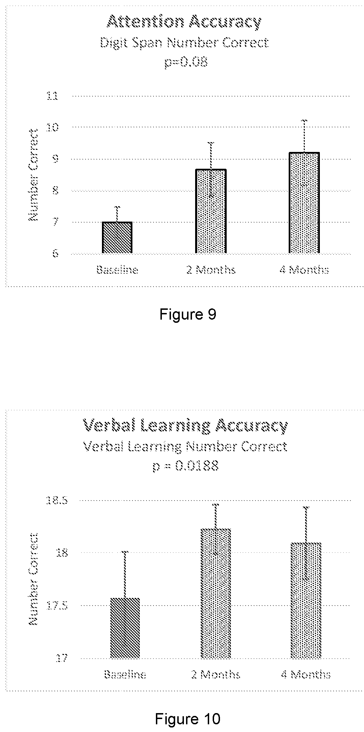

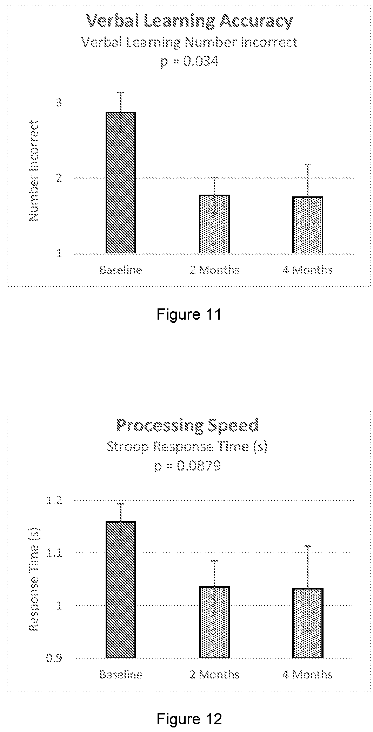

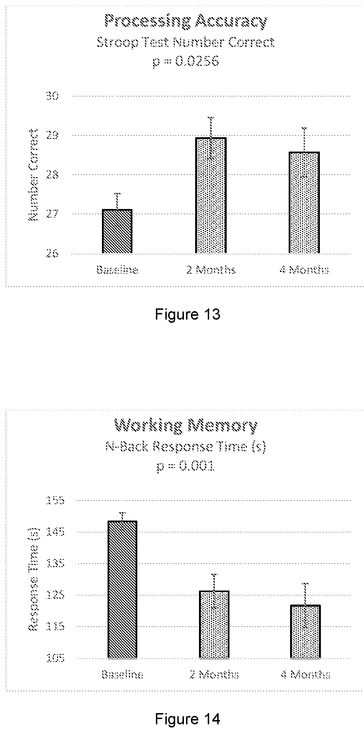

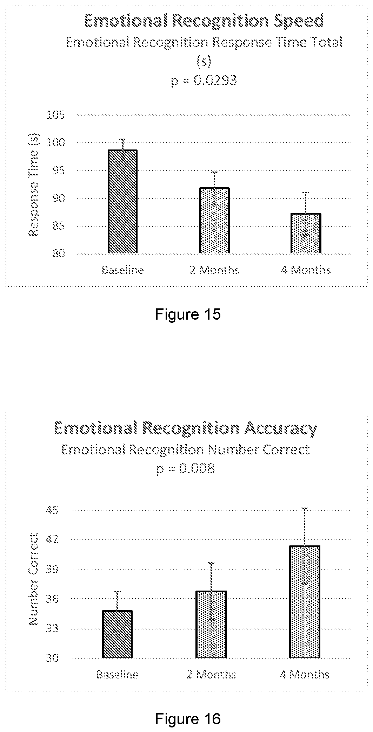

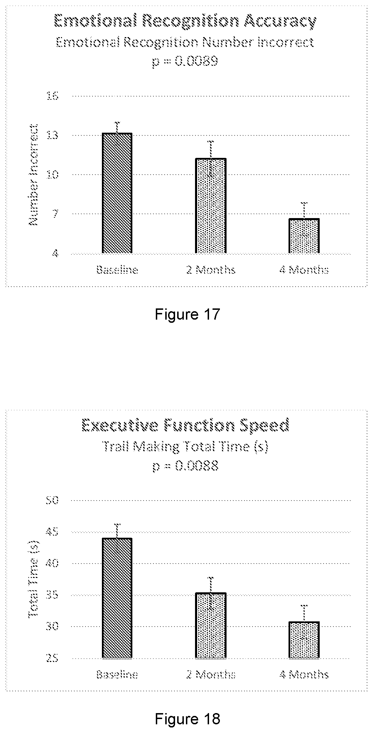

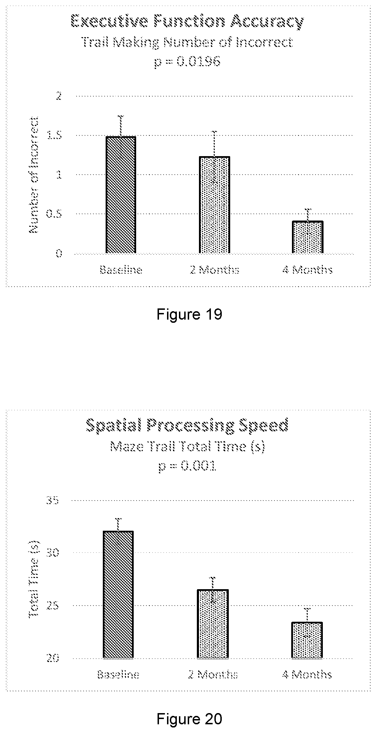

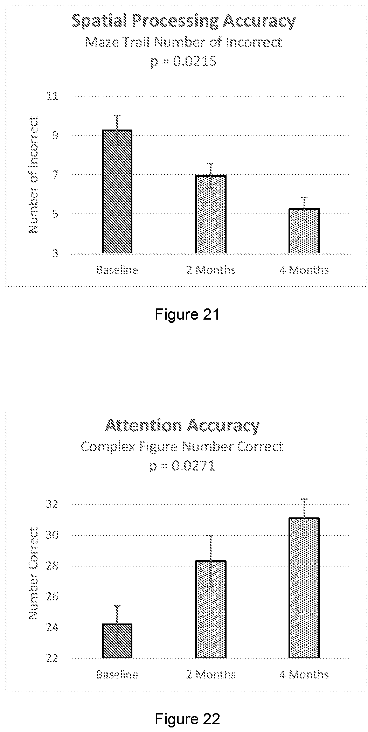

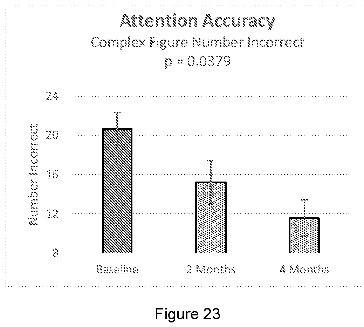

Two formulations (formulation 1 and formulation 2) were tested to assess their effects on various aspects of human cognition. These tests are well known to those skilled in the art and described generally in references 72-104. Healthy adults between the ages of 30 and 80 were administered a plurality of tests every 8 weeks to measure cognitive health and mental performance. Formulation 1 was administered to 15 participants with an average age of 44. Formulation 2 was administered to 26 participants with an average age of 62. The results of tests of formulation 1 is presented in Figures 1-9. The results of tests of formulation 2 are presented in Figures 10-23.

Formulation 1: A) Vitamin E (d-alpha-tocopherol succinate (1,210IU/g)) Concentration / Pill: 83mg (100IU); B) Alpha-Lipoic Acid (99%) Concentration / Pill: 50 mg; C) *Centella asiatica* Extract - Whole Plant, Concentration / Pill: 200 mg - (20% Asiaticosides ~40 mg); D) Panax Ginseng Extract - Root Extract Concentration / Pill: 175 mg - (Std. to 5% Ginsenosides ~8.75 mg); E) Selenium (L-Selenomethionine) 0.5% Se, Concentration / Pill: 25 mcg. Suggested dosage: 2 pills/capsules per day, preferably with food taken together in the morning. Pills/capsules may comprise other constituents as would be understood by a person of skill in the art, for example, but not limited to microcrystalline cellulose, magnesium stearate, silicon dioxide and the like.

Formulation 2 (not claimed): A) Vitamin E (d-alpha-tocopherol succinate (1,210IU/g)) Concentration / Pill: 125mg (150IU); B) Alpha-Lipoic Acid (99%) Concentration / Pill: 100 mg; C) *Centella asiatica* Extract - Whole Plant, Concentration / Pill: 175 mg - (20% Asiaticosides ~35 mg); D) Panax Ginseng Extract - Stems & Leaves Concentration / Pill: 150 mg - (Std. to 5% Ginsenosides ~7.5 mg); E) Selenium (L-Selenomethionine) 0.5% Se, Concentration / Pill: 25 mcg. Suggested dosage: 2 pills/capsules per day, preferably with food, taken together in the morning.

The results of the tests presented in Figures 1-23 suggest that the compositions/formulations as described herein can be used to enhance and/or increase spatial processing and memory, working memory, executive function, multi-tasking, verbal learning, emotional recognition, visuospatial processing or any combination thereof in subjects. Further, the results of the tests presented in Figures 1-23 suggest that the compositions/formulations as described herein can be used to prevent decline in spatial processing and memory, working memory, executive function, multi-tasking, verbal learning, emotional recognition, visuospatial processing or any combination thereof in subjects.

In addition to the results presented in the figures and as described above, subjects administered the formulations noted perceived increases in or more of: short and long-term memory, increased ability to learn new tasks, better quality sleep, higher energy levels, enhanced mood, enhanced ability to focus, decreased stress levels, increased confidence, better drive, less migraines, and reduced cognitive impairment from lack of sleep.

The specific formulations and compositions described herein also exhibit novel features and characteristics in terms of the individual components in the composition, their amounts, process by which they are obtained and their dosage recommendation. For example, but not to be considered limiting in any manner, ginseng root extract is preferred over ginseng stem and leaf extract for the reduction of adverse effects, such as, but not limited to indigestion in subjects. Similarly, formulations comprising high concentrations of asiaticosides, for example 40% asiaticoside concentration was associated with the adverse effect of lucid dreams within subjects. Formulations comprising 20% w/w asiaticoside concentrations (or less) is associated with a significantly decreased risk of such an adverse effect. Also, formulations taken on an empty stomach result in greater instances of acid reflux and/or indigestion in subject populations. Thus, it is preferable that the formulations be taken with food. Additionally, when taken at night, the formulations can cause wakefulness and an inability to sleep. Thus, it is preferable that the formulations be taken in the morning only.

### References

1. Choi, D. W. Calcium-mediated neurotoxicity: relationship to specific channel types and role in ischemic damage. Trends in neurosciences 11, 465-469 (1988).
2. Choi, J. H. & Yu, B. P. Brain synaptosomal aging: free radicals and membrane fluidity. Free radical biology & medicine 18, 133-139 (1995).
3. Mattson, M. P. Calcium and neurodegeneration. Aging cell 6, 337-350, doi:10.1111/j.1474-9726.2007.00275.x (2007).
4. Lopez, N., Tormo, C., De Blas, I., Llinares, I. & Alom, J. Oxidative stress in Alzheimer's disease and mild cognitive impairment with high sensitivity and specificity. Journal of Alzheimer's disease : JAD 33, 823-829, doi:10.3233/JAD-2012-121528 (2013).
5. Sanchez-Mejia, R. O. & Mucke, L. Phospholipase A2 and arachidonic acid in Alzheimer's disease. Biochimica et biophysica acta 1801, 784-790, doi:10.1016/j.bbalip.2010.05.013 (2010).
6. Sanchez-Mejia, R. O. et al. Phospholipase A2 reduction ameliorates cognitive deficits in a mouse model of Alzheimer's disease. Nature neuroscience 11, 1311-1318, doi:10.1038/nn.2213 (2008).
7. Watson, S. N., Lee, J. R., Risling, T. E., Hermann, P. M. & Wildering, W. C. Diminishing glutathione availability and age-associated decline in neuronal excitability. Neurobiology of aging 35, 1074-1085, doi:10.1016/j.neurobiolaging.2013.11.007 (2014).
8. Watson, S. N., Nelson, M. A. & Wildering, W. C. Redox agents modulate neuronal activity and reproduce physiological aspects of neuronal aging. Neurobiology of aging 33, 149-161, doi:10.1016/j.neurobiolaging.2010.01.017 (2012).
9. Watson, S. N., Risling, T. E., Hermann, P. M. & Wildering, W. C. Failure of delayed nonsynaptic neuronal plasticity underlies age-associated long-term associative memory impairment. BMC neuroscience 13.
10. Watson, S. N., Wright, N., Hermann, P. M. & Wildering, W. C. Phospholipase A(2): the key to reversing long-term memory impairment in a gastropod model of aging. Neurobiology of aging 34, 610-620, doi:10.1016/j.neurobiolaging.2012.02.028 (2013).
11. Sultana, R. & Butterfield, D. A. Role of oxidative stress in the progression of Alzheimer's disease. Journal of Alzheimer's disease : JAD 19, 341-353, doi:0U88727833343HV6.
12. Whalley, L. J., Deary, I. J., Appleton, C. L. & Starr, J. M. Cognitive reserve and the neurobiology of cognitive aging. Ageing research reviews 3, 369-382, doi:DOI 10.1016/j.arr.2004.05.001 (2004).
13. Cini, M. & Moretti, A. Studies on lipid peroxidation and protein oxidation in the aging brain. Neurobiology of aging 16, 53-57 (1995).
14. Gamper, N. et al. Oxidative modification of M-type K(+) channels as a mechanism of cytoprotective neuronal silencing. The EMBO journal 25, 4996-5004, doi:10.1038/sj.emboj.7601374 (2006).
15. Hool, L. C. & Corry, B. Redox control of calcium channels: from mechanisms to therapeutic opportunities. Antioxidants & redox signaling 9, 409-435, doi:10.1089/ars.2006.1446 (2007).
16. Spiteller, G. The important role of lipid peroxidation processes in aging and age dependent diseases. Molecular biotechnology 37, 5-12 (2007).
17. Lee, J. C., Simonyi, A., Sun, A. Y. & Sun, G. Y. Phospholipases A2 and neural membrane dynamics: implications for Alzheimer's disease. Journal of neurochemistry 116, 813-819, doi:10.1111/j.1471-4159.2010.07033.x (2011).
18. Choe, M., Jackson, C. & Yu, B. P. Lipid peroxidation contributes to age-related membrane rigidity. Free radical biology & medicine 18, 977-984 (1995).
19. Yu, B. P., Suescun, E. A. & Yang, S. Y. Effect of age-related lipid peroxidation on membrane fluidity and phospholipase A2: modulation by dietary restriction. Mechanisms of ageing and development 65, 17-33 (1992).
20. Greilberger, J. et al. Malondialdehyde, carbonyl proteins and albumin-disulphide as useful oxidative markers in mild cognitive impairment and Alzheimer's disease. Free radical research 42, 633-638, doi:10.1080/10715760802255764 (2008).
21. Jain, S. K., Ross, J. D., Levy, G. J., Little, R. L. & Duett, J. The accumulation of malonyldialdehyde, an end product of membrane lipid peroxidation, can cause potassium leak in normal and sickle red blood cells. Biochemical medicine and metabolic biology 42, 60-65 (1989).
22. Keller, J. N. et al. Evidence of increased oxidative damage in subjects with mild cognitive impairment. Neurology 64, 1152-1156, doi:10.1212/01.WNL.0000156156.13641.BA (2005).
23. Butterfield, D. A., Bader Lange, M. L. & Sultana, R. Involvements of the lipid peroxidation product, HNE, in the pathogenesis and progression of Alzheimer's disease. Biochimica et biophysica acta 1801, 924-929, doi:10.1016/j.bbalip.2010.02.005 (2010).
24. Butterfield, D. A. & Lauderback, C. M. Lipid peroxidation and protein oxidation in Alzheimer's disease brain: potential causes and consequences involving amyloid beta-peptide-associated free radical oxidative stress. Free radical biology & medicine 32, 1050-1060 (2002).
25. Gray, D. A. & Woulfe, J. Lipofuscin and aging: a matter of toxic waste. Sci Aging Knowledge Environ 2005, re1, doi:2005/5/re1.
26. Markesbery, W. R. Oxidative stress hypothesis in Alzheimer's disease. Free radical biology & medicine 23, 134-147 (1997).
27. Terman, A. Garbage catastrophe theory of aging: imperfect removal of oxidative damage? Redox report : communications in free radical research 6, 15-26 (2001).
28. Terman, A. & Brunk, U. T. Oxidative stress, accumulation of biological 'garbage', and aging. Antioxidants & redox signaling 8, 197-204, doi:10.1089/ars.2006.8.197 (2006).
29. Muralikrishna Adibhatla, R. & Hatcher, J. F. Phospholipase A2, reactive oxygen species, and lipid peroxidation in cerebral ischemia. Free radical biology & medicine 40, 376-387, doi: S0891-5849(05)00521-6.
30. Nigam, S. & Schewe, T. Phospholipase A(2)s and lipid peroxidation. Biochimica et biophysica acta 1488, 167-181 (2000).
31. Hermann, P. M., Watson, S. N. & Wildering, W. C. Phospholipase A2 - nexus of aging, oxidative stress, neuronal excitability, and functional decline of the aging nervous system? Insights from a snail model system of neuronal aging and age-associated memory impairment. Front Genet 5, 419, doi:10.3389/fgene.2014.00419 (2014).
32. Villarroel, A. & Schwarz, T. L. Inhibition of the Kv4 (Shal) family of transient K+ currents by arachidonic acid. The Journal of neuroscience : the official journal of the Society for Neuroscience 16, 2522-2532 (1996).
33. Angelova, P. & Muller, W. Oxidative modulation of the transient potassium current IA by intracellular arachidonic acid in rat CA1 pyramidal neurons. The European journal of neuroscience 23, 2375-2384, doi:10.1111/j.1460-9568.2006.04767.x (2006).
34. Colbert, C. M. & Pan, E. Arachidonic acid reciprocally alters the availability of transient and sustained dendritic K(+) channels in hippocampal CA1 pyramidal neurons. The Journal of neuroscience : the official journal of the Society for Neuroscience 19, 8163-8171 (1999).
35. Boland, L. M. & Drzewiecki, M. M. Polyunsaturated fatty acid modulation of voltage-gated ion channels. Cell biochemistry and biophysics 52, 59-84, doi:10.1007/s12013-008-9027-2 (2008).
36. Denson, D. D., Wang, X., Worrell, R. T. & Eaton, D. C. Effects of fatty acids on BK channels in GH(3) cells. Am J Physiol Cell Physiol 279, C1211-1219 (2000).
37. Holmqvist, M. H. et al. Kinetic modulation of Kv4-mediated A-current by arachidonic acid is dependent on potassium channel interacting proteins. The Journal of neuroscience : the official journal of the Society for Neuroscience 21, 4154-4161 (2001).
38. Ruppersberg, J. P., Frank, R., Pongs, O. & Stocker, M. Cloned neuronal IK(A) channels reopen during recovery from inactivation. Nature 353, 657-660, doi:10.1038/353657a0 (1991).
39. Danthi, S. J., Enyeart, J. A. & Enyeart, J. J. Modulation of native T-type calcium channels by omega-3 fatty acids. Biochemical and biophysical research communications 327, 485-493, doi:10.1016/j.bbrc.2004.12.033 (2005).
40. Schmitt, H. & Meves, H. Modulation of neuronal calcium channels by arachidonic acid and related substances. The Journal of membrane biology 145, 233-244 (1995).
41. Liu, L. & Rittenhouse, A. R. Effects of arachidonic acid on unitary calcium currents in rat sympathetic neurons. The Journal of physiology 525 Pt 2, 391-404 (2000).
42. Atkinson, J., Epand, R. F. & Epand, R. M. Tocopherols and tocotrienols in membranes: a critical review. Free radical biology & medicine 44, 739-764, doi:10.1016/j.freeradbiomed.2007.11.010 (2008).
43. Kagan, V. E. Tocopherol Stabilizes Membrane against Phospholipase-a, Free Fatty-Acids, and Lysophospholipids. Annals of the New York Academy of Sciences 570, 121-135, doi:DOI 10.1111/j.1749-6632.1989.tb14913.x (1989).
44. Erin, A. N., Skrypin, V. V. & Kagan, V. E. Formation of alpha-tocopherol complexes with fatty acids. Nature of complexes. Biochimica et biophysica acta 815, 209-214 (1985).
45. Erin, A. N., Spirin, M. M., Tabidze, L. V. & Kagan, V. E. Formation of alpha-tocopherol complexes with fatty acids. A hypothetical mechanism of stabilization of biomembranes by vitamin E. Biochimica et biophysica acta 774, 96-102 (1984).
46. Mukherjee, A. K., Ghosal, S. K. & Maity, C. R. Lysosomal membrane stabilization by alpha-tocopherol against the damaging action of Vipera russelli venom phospholipase A2. Cellular and molecular life sciences : CMLS 53, 152-155 (1997).
47. Sevanian, A. & Kim, E. Phospholipase A2 dependent release of fatty acids from peroxidized membranes. Journal of free radicals in biology & medicine 1, 263-271 (1985).
48. Sevanian, A., Muakkassah-Kelly, S. F. & Montestruque, S. The influence of phospholipase A2 and glutathione peroxidase on the elimination of membrane lipid peroxides. Archives of biochemistry and biophysics 223, 441-452 (1983).
49. Tan, K. H., Meyer, D. J., Belin, J. & Ketterer, B. Inhibition of microsomal lipid peroxidation by glutathione and glutathione transferases B and AA. Role of endogenous phospholipase A2. The Biochemical journal 220, 243-252 (1984).
50. Espinoza, S. E. et al. Glutathione peroxidase enzyme activity in aging. The journals of gerontology. Series A, Biological sciences and medical sciences 63, 505-509 (2008).
51. Zhu, Y., Carvey, P. M. & Ling, Z. Age-related changes in glutathione and glutathione-related enzymes in rat brain. Brain research 1090, 35-44, doi:10.1016/j.brainres.2006.03.063 (2006).
52. Xia, X. et al. Anti-tumor activity of three novel a derivative of ginsenoside on colorectal cancer cells. Steroids 80, 24-29, doi:10.1016/j.steroids.2013.11.018 (2014).
53. Rastogi, V., Santiago-Moreno, J. & Dore, S. Ginseng: a promising neuroprotective strategy in stroke. Frontiers in cellular neuroscience 8, 457, doi:10.3389/fncel.2014.00457 (2014).
54. Uzayisenga, R., Ayeka, P. A. & Wang, Y. Anti-diabetic potential of Panax notoginseng saponins (PNS): a review. Phytotherapy research : PTR 28, 510-516, doi:10.1002/ptr.5026 (2014).
55. Yu, J. et al. Antioxidative effect of ginseng stem-leaf saponins on oxidative stress induced by cyclophosphamide in chickens. Poultry science, doi:10.3382/ps/pev055 (2015).
56. Bak, M. J., Jeong, W. S. & Kim, K. B. Detoxifying effect of fermented black ginseng on H2O2-induced oxidative stress in HepG2 cells. International journal of molecular medicine 34, 1516-1522, doi:10.3892/ijmm.2014.1972 (2014).
57. Choi HJ, H. H., Park JH, Son JH, Bae JH, Seung TS. Antioxidantive, phospholipase A2 inhibiting, and anticancer effect of polyphenol rich fractions from Panax ginseng C. A. Meyer. J Korean Soc Agric Chem Biotechnol 46, 251-256 (2003).
58. Aoyama, K., Watabe, M. & Nakaki, T. Regulation of neuronal glutathione synthesis. Journal of pharmacological sciences 108, 227-238 (2008).
59. Vaillancourt, F. et al. 4-Hydroxynonenal induces apoptosis in human osteoarthritic chondrocytes: the protective role of glutathione-S-transferase. Arthritis research & therapy 10, R107, doi:10.1186/ar2503 (2008).
60. Xie, C., Lovell, M. A. & Markesbery, W. R. Glutathione transferase protects neuronal cultures against four hydroxynonenal toxicity. Free radical biology & medicine 25, 979-988 (1998).
61. Ruffmann, R. & Wendel, A. GSH rescue by N-acetylcysteine. Klinische Wochenschrift 69, 857-862 (1991).
62. Kumar, P., Taha, A., Kale, R. K., Cowsik, S. M. & Baquer, N. Z. Physiological and biochemical effects of 17beta estradiol in aging female rat brain. Experimental gerontology 46, 597-605, doi:10.1016/j.exger.2011.02.008 (2011).
63. Liebler, D. C., Kling, D. S. & Reed, D. J. Antioxidant protection of phospholipid bilayers by alpha-tocopherol. Control of alpha-tocopherol status and lipid peroxidation by ascorbic acid and glutathione. The Journal of biological chemistry 261, 12114-12119 (1986).
64. Akiba, S. & Sato, T. Cellular function of calcium-independent phospholipase A2. Biological & pharmaceutical bulletin 27, 1174-1178 (2004).
65. McLean, L. R., Hagaman, K. A. & Davidson, W. S. Role of lipid structure in the activation of phospholipase A2 by peroxidized phospholipids. Lipids 28, 505-509 (1993).
66. Kim, S. S., Kim, D. K. & Suh, Y. H. Cerebral cortical phospholipase A2 activity of senescence-accelerated mouse is increased in an age-dependent manner. Neuroscience research 29, 269-272 (1997).
67. Stevens, J. F. & Maier, C. S. Acrolein: sources, metabolism, and biomolecular interactions relevant to human health and disease. Molecular nutrition & food research 52, 7-25, doi:10.1002/mnfr.200700412 (2008).
68. Veerendra Kumar, M. H. & Gupta, Y. K. Effect of Centella asiatica on cognition and oxidative stress in an intracerebroventricular streptozotocin model of Alzheimer's disease in rats. Clinical and experimental pharmacology & physiology 30, 336-342 (2003).
69. Defillipo, P. P. et al. Inhibition of cPLA2 and sPLA2 activities in primary cultures of rat cortical neurons by Centella asiatica water extract. Natural product communications 7, 841-843 (2012).
70. Watson, Shawn N., (2013). Lipid Peroxidation as an Exponent of Neuronal Senescence. Ph.D. Thesis. University of Calgary: Canada.
71. Defillipo, P. P., Raposo, A. H., Fedoce, A. G., Ferreira, A. S., Polonini, H. C., Gattaz, W. F., & Raposo, N. R. Inhibition of cPLA2 and sPLA2 activities in primary cultures of rat cortical neurons by Centella asiatica water extract. Nat. Prod. Comm., 7(7), 841-843, (2012).
72. Boake, C. (2000). Edouard Claparede and the auditory verbal learning test. Journal of Clinical and Experimental Neuropsychology, 22(2), 286-292.
73. Delaney, R. C., Prevey, M. L., Cramer, J., Mattson, R. H., & VA Epilepsy Cooperative Study #264 Research Group. (1992). Test-retest comparability and control subject data for the rey auditory verbal learning test and rey-osterrieth/taylor complex figures. Archives of Clinical Neuropsychology, 7(6), 523-528.
74. Powell, J. B., Cripe, L. I., & Dodrill, C. B. (1991). Assessment of brain impairment with the Rey Auditory Verbal Learning Test: A comparison with other neuropsychological measures. Archives of Clinical Neuropsychology, 6(4), 241-249.
75. Rey, A. (1964). L'examen clinique en psychologie [The clinical examination in psychology]. Oxford, England: Presses Universitaries De France.
76. Schmidt, M. (1996). Rey auditory verbal learning test: A handbook (p. 1996). Los Angeles, CA: Western Psychological Services.
77. Luria, A. R. (2012). Higher cortical functions in man. Springer Science & Business Media.
78. Dodrill, C. B. (1978). A neuropsychological battery for epilepsy, Epilepsia, 19, 611-623.
79. Everatt, J., Warner, J., Miles, T. R., & Thomson, M. E. (1997). The incidence of Stroop interference in dyslexia. Dyslexia, 3(4), 222-228.
80. Klein, G. S. (1964). Semantic power measured through the interference of words with colornaming. The American journal of psychology, 77(4), 576-588.
81. Mishra, J., Sagar, R., Joseph, A. A., Gazzaley, A., & Merzenich, M. M. (2016). Training sensory signal-to-noise resolution in children with ADHD in a global mental health setting. Translational psychiatry, 6(4), e781.
82. Stroop, J. R. (1935). Studies of interference in serial verbal reactions. Journal of experimental psychology, 18(6), 643.
83. Lezak, M. D. (2004). Neuropsychological assessment. Oxford University Press, USA.
84. Dobbs, A. R., & Rule, B. G. (1989). Adult age differences in working memory. Psychology and Aging, 4, 500-503.
85. Sweet, L. H. (2011). N-Back Paradigm. In Encyclopedia of Clinical Neuropsychology (pp. 1718-1719). Springer New York.
86. Jaeggi, S. M., Buschkuehl, M., Jonides, J., & Perrig, W. J. (2008). Improving fluid intelligence with training on working memory. Proceedings of the National Academy of Sciences, 105(19), 6829-6833.
87. Ekman, P., & Friesen, W. V. (1975). Unmasking the face: A guide to recognizing emotions from facial cues.
88. Hornak, J., Rolls, E. T., & Wade, D. (1996). Face and voice expression identification in patients with emotional and behavioural changes following ventral frontal lobe damage. Neuropsychologia, 34(4), 247-261.
89. Meyers, J. E. (2011). Trail Making Test. Encyclopedia of clinical neuropsychology, 2537-2538.
90. Reitan, R. M. (1986). Trail Making Test: Manual for administration and scoring. Reitan Neuropsychology Laboratory.
91. Snyder, H. R. (2013). Major depressive disorder is associated with broad impairments on neuropsychological measures of executive function: A meta-analysis and review.
92. Milner, B. (1965). Visually-guided maze learning in man: Effects of bilateral hippocampal, bilateral frontal, and unilateral cerebral lesions. Neuropsychologia, 3(4), 317-338.
93. Porteus, S. D. (1965). Porteus Maze Tests: Fifty years' application.
94. Stern, R. A., & White, T. (2003). Neuropsychological Assessment Battery. Psychological Assessment Resources, Inc: Lutz, FL, USA.
95. Anderson, P. (2002). Assessment and development of executive function (EF) during childhood. Child neuropsychology, 8(2), 71-82.
96. Berry, D. T. R., Allen, R. S., & Schmitt, F. A. (1991). Rey-Osterrieth complex figure: Psychometric characteristics in a geriatric sample. The Clinical Neuropsychologist, 5(2), 143-153.
97. Bigler, E. D., Rosa, L., Schultz, F., Hall, S., & Harris, J. (1989). Rey- Auditory Verbal Learning and Rey- Osterrieth Complex Figure Design performance in Alzheimer's disease and closed head injury. Journal of clinical psychology, 45(2), 277-280.
98. Osterrieth, P. A. (1944). Le test de copie d'une figure complexe; contribution a l'etude de la perception et de la memoire. Archives de psychologie.
99. Rey, A. (1964). L'examen clinique en psychologie. Paris: Presses Univeritaires de France. Topiwala, A., Allan, C. L., Valkanova, V., Zsoldos, E., Filippini, N., Sexton, C. & Kivimäki, M. (2017). Moderate alcohol consumption as risk factor for adverse brain outcomes and cognitive decline: longitudinal cohort study. bmj, 357, j2353.
100. Etherton, J. L., Bianchini, K. J., Greve, K. W., & Heinly, M. T. (2005). Sensitivity and specificity of reliable digit span in malingered pain-related disability. Assessment, 12(2), 130-136.
101. Low, M., Farrell, A., Biggs, B. A., & Pasricha, S. R. (2013). Effects of daily iron supplementation in primary-school-aged children: systematic review and meta-analysis of randomized controlled trials. Canadian Medical Association Journal, cmaj-130628.
102. Rudel, R. G., & Denckla, M. B. (1974). Relation of forward and backward digit repetition to neurological impairment in children with learning disabilities. Neuropsychologia, 12, 109-118.
103. Wechsler, D. (2014). Wechsler Adult Intelligence Scale-Fourth Edition (WAIS-IV).
104. Zuk, J., Benjamin, C., Kenyon, A., & Gaab, N. (2014). Behavioral and neural correlates of executive functioning in musicians and non-musicians. PloS one, 9(6), e99868.

## Claims

1. A composition in unit dose form comprising:
asiaticosides in an amount of 20-80 mg, wherein the asiaticosides are derived from *Centella asiatica* extract;
d-α-tocopherol succinate in an amount of 40-200 mg;
ginsenosides in an amount of 4-20 mg, wherein the ginsenosides are derived from Ginseng root extract;
L-selenomethionine in an amount of 10-50 mcg, and;
α-lipoic acid in an amount of 25-200 mg;
wherein the asiaticosides are present in an amount of 20% w/w or less of the composition.

2. The composition of claim 1 in a unit dosage form comprising 50 mg or 100 mg α-lipoic acid, 25 mcg L-selenomethionine, 83 mg or 125 mg d-α-tocopherol succinate, 200 mg or 175 mg *Centella asiatica* whole plant extract providing 40 mg or 35 mg asiaticosides, respectively; and 175 mg or 150 mg Panax Ginseng extract comprising 8.75 mg and 7.5 mg ginsenosides, respectively.

3. The composition of claim 1 further comprising one or more pharmaceutically acceptable excipients.

4. The composition of claim 3 wherein the one or more pharmaceutically acceptable excipients comprise microcrystalline cellulose, magnesium stearate, silicon dioxide or a combination thereof.

5. The composition of claim 1 for use in treating and/or preventing cognitive decline in a subject in need thereof.

6. The composition of claim 1 for use in treating or preventing cognitive decline in a subject in need thereof.

7. The composition for use according to claim 6 wherein the cognitive decline is normal age-related cognitive decline.

8. The composition for use according to claim 6, wherein the cognitive decline is loss of cognitive function, loss of memory, memory impairment, loss of short term memory, loss of intermediate term memory, loss of long term memory or loss of an ability to learn, store or recall information, loss of attention span, loss of language skills, loss of writing skills or loss of problem solving skills.

9. The composition for use according to claim 8, wherein the cognitive decline is from a degenerative disease or disorder.

10. The composition for use according to claim 9, wherein the degenerative disease or disorder is Alzheimer's disease, Lewy body dementia, vascular dementia, Parkinson's disease-associated dementia, Huntington's disease-associated dementia, AIDS-associated dementia, benign senile forgetfulness, Down's syndrome-associated dementia, multi-infarct dementia, multiple sclerosis, tardive dyskinesia, Wernicke-Korsakoff syndrome or alcoholism-associated dementia.

11. A method of making the composition of claim 1 comprising,
combining d-alpha-tocopherol succinate, alpha-lipoic acid, *centella asiatica* extract, panax ginseng root extract and L-selenomethionine, wherein *centella asiatica* extract and panax ginseng root extract are derived from water-ethanol extractions.

12. The method of claim 11, further comprising packaging the composition in a capsule with microcrystalline cellulose, magnesium stearate and silicon dioxide.

## Patentansprüche

1. Zusammensetzung in Einheitsdosisform, umfassend:
Asiaticoside in einer Menge von 20-80 mg, wobei die Asiaticoside von *Centella asiatica-Extrakt* stammen;
d-α-Tocopherolsuccinat in einer Menge von 40-200 mg;
Ginsenoside in einer Menge von 4-20 mg, wobei die Ginsenoside von Ginsengwurzel-Extrakt stammen;
L-Selenomethionin in einer Menge von 10-50 µg und
α-Liponsäure in einer Menge von 25-200 mg;
wobei die Asiaticoside in einer Menge von 20 Gew.-% der Zusammensetzung oder weniger vorhanden sind.

2. Zusammensetzung nach Anspruch 1 in einer Einheitsdarreichungsform, umfassend 50 mg oder 100 mg α-Liponsäure, 25 µg L-Selenomethionin, 83 mg oder 125 mg d-α-Tocopherolsuccinat, 200 mg oder 175 mg *Centella* asiatica-Ganzpflanze-Extrakt, der 40 mg bzw. 35 mg Asiaticoside liefert; und 175 mg oder 150 mg Panax-Ginseng-Extrakt, der 8,75 mg bzw. 7,5 mg Ginsenoside umfasst.

3. Zusammensetzung nach Anspruch 1, ferner umfassend einen oder mehrere pharmazeutisch unbedenkliche Exzipienten.

4. Zusammensetzung nach Anspruch 3, wobei die ein oder mehreren pharmazeutisch unbedenklichen Exzipienten mikrokristalline Cellulose, Magnesiumstearat, Siliciumdioxid oder eine Kombination davon umfassen.

5. Zusammensetzung nach Anspruch 1 zur Verwendung bei der Behandlung und/oder Vorbeugung von kognitivem Abbau bei einem behandlungsbedürftigen Individuum.

6. Zusammensetzung nach Anspruch 1 zur Verwendung bei der Behandlung oder Vorbeugung von kognitivem Abbau bei einem behandlungsbedürftigen Individuum.

7. Zusammensetzung zur Verwendung gemäß Anspruch 6, wobei es sich bei dem kognitiven Abbau um normalen altersbedingten kognitiven Abbau handelt.

8. Zusammensetzung zur Verwendung gemäß Anspruch 6, wobei es sich bei dem kognitiven Abbau um kognitiven Funktionsverlust, Gedächtnisverlust, Gedächtnisstörung, Verlust des Kurzzeitgedächtnisses, Verlust des mittelfristigen Gedächtnisses, Verlust des Langzeitgedächtnisses oder Verlust einer Fähigkeit zum Lernen, Speichern oder Abrufen von Informationen, Verlust der Aufmerksamkeitsspanne, Verlust von Sprachkenntnissen, Verlust von Schreibkenntnissen oder Problemlösungsfähigkeitsverlust handelt.

9. Zusammensetzung zur Verwendung gemäß Anspruch 8, wobei der kognitive Abbau von einer degenerativen Krankheit oder Störung herrührt.

10. Zusammensetzung zur Verwendung gemäß Anspruch 9, wobei es sich bei der degenerativen Krankheit oder Störung um Morbus Alzheimer, Lewy-Körperchen-Demenz, vaskuläre Demenz, mit Morbus Parkinson assoziierte Demenz, mit Chorea Huntington assoziierte Demenz, mit AIDS assoziierte Demenz, milde Altersvergesslichkeit, mit Down-Syndrom assoziierte Demenz, Multi-Infarkt-Demenz, multiple Sklerose, Dyskinesia tarda, Wernicke-Korsakoff-Syndrom oder mit Alkoholismus assoziierte Demenz handelt.

11. Verfahren zur Herstellung der Zusammensetzung nach Anspruch 1, umfassend
Kombinieren von d-alpha-Tocopherolsuccinat, alpha-Liponsäure, *Centella-asiatica-Extrakt,* Panax-Ginsengwurzel-Extrakt und L-Selenomethionin, wobei *Centella-asiatica-Extrakt* und Panax-Ginsengwurzel-Extrakt von Wasser-Ethanol-Extraktionen stammen.

12. Verfahren nach Anspruch 11, ferner umfassend Verpacken der Zusammensetzung in einer Kapsel mit mikrokristalliner Cellulose, Magnesiumstearat und Siliciumdioxid.

## Revendications

1. Composition sous forme posologique unitaire comprenant :
des asiaticosides en une quantité de 20-80 mg, les asiaticosides étant dérivés d'un extrait de *Centella asiatica ;*
du succinate de d-α-tocophérol en une quantité de 40-200 mg ;
des ginsénosides en une quantité de 4-20 mg, dans laquelle les ginsénosides sont dérivés d'un extrait de racine de ginseng ;
de la L-sélénométhionine en une quantité de 10-50 µg, et ;
de l'acide α-lipoïque en une quantité de 25-200 mg ;
dans laquelle les asiaticosides sont présents en une quantité de 20 % p/p ou moins de la composition.

2. Composition selon la revendication 1 sous une forme posologique unitaire comprenant 50 mg ou 100 mg d'acide α-lipoïque, 25 µg de L-sélénométhionine, 83 mg ou 125 mg de succinate de d-α-tocophérol, 200 mg ou 175 mg d'extrait de plante entière de *Centella asiatica* fournissant respectivement 40 mg ou 35 mg d'asiaticosides ; et 175 mg ou 150 mg d'extrait de Panax ginseng comprenant 8,75 mg et 7,5 mg de ginsénosides, respectivement.

3. Composition selon la revendication 1, comprenant en outre un ou plusieurs excipients pharmaceutiquement acceptables.

4. Composition selon la revendication 3, dans laquelle l'excipient ou les excipients pharmaceutiquement acceptables comprennent de la cellulose microcristalline, du stéarate de magnésium, du dioxyde de silicium ou une combinaison de ceux-ci.

5. Composition selon la revendication 1, destinée à être utilisée dans le traitement et/ou la prévention du déclin cognitif chez un sujet qui en a besoin.

6. Composition selon la revendication 1, destinée à être utilisée dans le traitement ou la prévention du déclin cognitif chez un sujet qui en a besoin.

7. Composition pour utilisation selon la revendication 6, dans laquelle le déclin cognitif est un déclin cognitif lié à l'âge normal.

8. Composition pour utilisation selon la revendication 6, dans laquelle le déclin cognitif est une perte de fonction cognitive, une perte de mémoire, une altération de la mémoire, une perte de mémoire à court terme, une perte de mémoire à moyen terme, une perte de mémoire à long terme ou une perte d'une capacité d'apprentissage, de stockage ou de rappel d'informations, une perte d'attention, une perte de compétences linguistiques, une perte de compétences en écriture ou une perte de capacité de résolution de problèmes.

9. Composition pour utilisation selon la revendication 8, dans laquelle le déclin cognitif provient d'une maladie dégénérative ou d'un trouble dégénératif.

10. Composition pour utilisation selon la revendication 9, dans laquelle la maladie dégénérative ou le trouble dégénératif est la maladie d'Alzheimer, la démence à corps de Lewy, la démence vasculaire, la démence associée à la maladie de Parkinson, la démence associée à la maladie de Huntington, la démence associée au sida, l'oubli sénile bénin, la démence associée au syndrome de Down, la démence à infarctus multiples, la sclérose en plaques, la dyskinésie tardive, le syndrome de Wernicke-Korsakoff ou la démence associée à l'alcoolisme.

11. Procédé de préparation de la composition selon la revendication 1, comprenant,
la combinaison de succinate de d-alpha-tocophérol, d'acide alpha-lipoïque, d'extrait de *centella asiatica,* d'extrait de racine de panax ginseng et de L-sélénométhionine, dans lequel l'extrait de *centella asiatica* et l'extrait de racine de panax ginseng sont dérivés d'extractions à l'eau-éthanol.

12. Procédé selon la revendication 11, comprenant en outre le conditionnement de la composition dans une capsule avec de la cellulose microcristalline, du stéarate de magnésium et du dioxyde de silicium.
